# EUROPÄISCHE PATENTANMELDUNG

(11) **EP 3 413 073 A1**
(43) Veröffentlichungstag der Anmeldung: **12.12.2018**
(21) Anmeldenummer: 17174697.7
(22) Anmeldetag: 07.06.2017
(51) Int. Cl.: G01R 33/56, G01R 33/563

(54) **KONTRASTMITTELUNTERSTÜTZTE MR-ANGIOGRAPHIE MIT ERMITTLUNG DER FLUSSGESCHWINDIGKEIT DES KONTRASTMITTELS**

(71) Anmelder: Siemens Healthcare GmbH, 91052 Erlangen (DE)
(72) Erfinder: Keil, Miriam, 91056 Erlangen-Dechsendorf (DE)

(57) **Zusammenfassung**

Die Erfindung betrifft ein Verfahren zur Durchführung einer angiographischen Messung von einem Hauptmessbereich eines Patienten mittels einer Magnetresonanzanlage sowie die zugehörige Magnetresonanzanlage und ein zugehöriges Computerprogrammprodukt.

Das erfindungsgemäße Verfahren zur Durchführung einer angiographischen Messung von einem Hauptmessbereich eines Patienten mittels einer Magnetresonanzanlage umfasst die folgenden Schritte:
- Durchführen zumindest einer Übersichtsmessung mittels der Magnetresonanzanlage, wobei Übersichtsmessdaten generiert werden,
- Festlegen des Hauptmessbereichs und eines ersten Messbereichs, welcher sich vom Hauptmessbereich unterscheidet, mittels der Übersichtsmessdaten,
- Durchführen einer ersten zeitaufgelösten Messung mittels der Magnetresonanzanlage in dem festgelegten ersten Messbereich, wobei erste zeitaufgelöste Messdaten generiert werden,
- Erfassen eines injizierten Kontrastmittelbolus in dem festgelegten ersten Messbereich mittels der ersten zeitaufgelösten Messdaten,
- Ermitteln einer Flussgeschwindigkeit des erfassten injizierten Kontrastmittelbolus,
- Festlegen zumindest eines Messparameters der angiographischen Messung gemäß der ermittelten Flussgeschwindigkeit und
- Durchführung der angiographischen Messung des Hauptmessbereichs des Patienten mit dem festgelegten zumindest einen Messparameter in der Magnetresonanzanlage.

## Beschreibung

Die Erfindung betrifft ein Verfahren zur Durchführung einer angiographischen Messung von einem Hauptmessbereich eines Patienten mittels einer Magnetresonanzanlage sowie die zugehörige Magnetresonanzanlage und ein zugehöriges Computerprogrammprodukt.

In einer Magnetresonanzanlage wird üblicherweise ein zu untersuchender Körper einer Untersuchungsperson, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, insbesondere Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Messdaten können schließlich die gewünschten Bilder rekonstruiert werden.

Eine angiographische Messung in einer Magnetresonanzanlage ist eine bekannte Untersuchungsmethode zur Darstellung des arteriellen und/oder venösen Gefäßsystems. Das Festlegen der Messparameter, insbesondere des zeitlichen Ablaufs, der angiographischen Messung erfordert besondere Aufmerksamkeit. Ein Grund hierfür ist, dass für die angiographische Messung ein Kontrastmittel injiziert wird, welches das Signal der Blutgefäße gegenüber dem Signal des umgebenden Gewebes verstärkt, und dass die Verteilung des Kontrastmittels im Körper erfasst werden soll. Üblicherweise werden Gadolinium-haltige Kontrastmittel injiziert. Durch Akquisition von Messdaten vor und nach der Injektion des Kontrastmittels kann mittels der Subtraktion der von den Messdaten rekonstruierten Bilder das umgebende Gewebe fast vollständig eliminiert werden. Bei der angiographischen Messung ist es daher erforderlich, die angiographische Messung zu einem optimalen Zeitpunkt nach der Injektion des Kontrastmittelbolus zu starten, um einen optimalen Bildkontrast zu erreichen.

Bisher wurden zur optimalen Festlegung der Messparameter der angiographischen Messung verschiedene Verfahren verwendet: Bei einem ersten Verfahren wird einem Patienten zunächst ein Pre-Kontrastmittelbolus, welcher eine geringe Menge (beispielsweise 1 mL) an Kontrastmittel umfasst, injiziert. Simultan wird ein Gefäß in der Nähe des zu untersuchenden Hauptmessbereichs mit einer zeitaufgelösten Messung dargestellt. Durch eine Auswertung des Signalverlaufs, insbesondere des Kontrastes innerhalb einer Gefäßstruktur über die Zeit können Charakteristika des Pre-Kontrastmittelbolus ermittelt werden. Davon können Rückschlüsse über Charakteristika eines Haupt-Kontrastmittelbolus, welcher eine übliche Menge (beispielsweise 8 mL) an Kontrastmittel aufweist, gezogen werden und dementsprechend können die Messparameter der angiographischen Messung festgelegt werden.

Bei diesem ersten Verfahren muss dem Patienten üblicherweise zweimal Kontrastmittel injiziert werden. Außerdem müssen die Rückschlüsse geeignet gezogen werden: so müssen die unterschiedlichen Charakteristika, insbesondere die unterschiedliche Anreicherung zwischen dem 1 mL-Kontrastmittelbolus und dem 8 mL-Kontrastmittelbolus von einem Benutzer interpretiert werden, so dass die Messparameter, insbesondere der Messstartzeitpunkt, der angiographischen Messung, geeignet festgelegt werden können.

Bei einem zweiten Verfahren wird ein Messbereich nahe des zu untersuchenden Hauptmessbereichs kontinuierlich mit einer zeitaufgelösten Messung gemessen. Insbesondere wird ein Gefäß, durch welches ein injizierter Kontrastmittelbolus zuerst fließt, mit der zeitaufgelösten Messung beobachtet, während der Kontrastmittelbolus injiziert wird. Sobald der Kontrastmittelbolus in dem Gefäß erfasst wird, wird die zeitaufgelöste Messung beendet und die angiographische Messung gestartet.

Dieses zweite Verfahren ist in der Durchführung wesentlich einfacher als das erste Verfahren, erfordert aber, dass die Messparameter der angiographischen Messung bereits im Vorfeld richtig eingestellt werden, so dass die angiographische Messung quasi unmittelbar gestartet werden kann. Wichtig ist beispielsweise hierbei, dass sich eine angenommene Kreislaufzeit des Patienten sich nicht auf Grund von Pathologien oder anderen Befunden stark von den "Standard"-Kreislaufzeiten unterscheiden, welche für die Parametrisierung der nachfolgenden angiographischen Messung angenommen werden. Die Kreislaufzeit erlaubt beispielsweise einen Rückschluss auf eine Flussgeschwindigkeit des Kontrastmittelbolus oder die Blutflussgeschwindigkeit.

Aus DE 10 2013 220 288 B4 ist ein Verfahren zum Aufnehmen von Magnetresonanz-Bilddaten, eine Bilddatenaufnahmeeinheit, ein Magnetresonanzgerät und ein Computerprogrammprodukt bekannt, bei welchem Steuerbefehle für eine Magnetresonanzanlage optimiert und überprüft werden.

Die DE 10 2011 007 835 A1 beschreibt ein Verfahren zum Erstellen eines Magnetresonanz-Angiographiebildes von einer Gefäßstruktur eines Untersuchungsbereichs, bei dem Spins im Untersuchungsbereich durch Einstrahlen von zumindest einem HF-Sättigungspuls gesättigt werden und bei der anschließenden Magnetresonanz-Signalaufnahme für die Erstellung des Magnetresonanz-Angiographiebildes eine geringere Signalintensität haben als Spins, die von einer Hauptarterie über eine Zuführungsarterie in den Untersuchungsbereich einfließen und nicht durch den zumindest einen HF-Sättigungspuls gesättigt werden und eine gegenüber den gesättigten Spins wesentlich erhöhte Signalintensität haben.

Der Erfindung liegt die Aufgabe zu Grunde, ein Verfahren zur Durchführung einer flexiblen und/oder schnellen angiographischen Messung von einem Hauptmessbereich eines Patienten mittels einer Magnetresonanzanlage sowie die Magnetresonanzanlage und das Computerprogrammprodukt anzugeben.

Die Aufgabe wird durch die Merkmale des unabhängigen Anspruchs gelöst. Vorteilhafte Weiterbildungen sind in den abhängigen Ansprüchen angegeben.

Nachstehend wird die erfindungsgemäße Lösung der Aufgabe sowohl in Bezug auf die beanspruchte Magnetresonanzanlage als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Magnetresonanzanlage gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Das erfindungsgemäße Verfahren zur Durchführung einer angiographischen Messung von einem Hauptmessbereich eines Patienten mittels einer Magnetresonanzanlage umfasst die folgenden Schritte:
- Durchführen zumindest einer Übersichtsmessung mittels der Magnetresonanzanlage, wobei Übersichtsmessdaten generiert werden,
- Festlegen des Hauptmessbereichs und eines ersten Messbereichs, welcher sich vom Hauptmessbereich unterscheidet, mittels der Übersichtsmessdaten,
- Durchführen einer ersten zeitaufgelösten Messung mittels der Magnetresonanzanlage in dem festgelegten ersten Messbereich, wobei erste zeitaufgelöste Messdaten generiert werden,
- Erfassen eines injizierten Kontrastmittelbolus in dem festgelegten ersten Messbereich mittels der ersten zeitaufgelösten Messdaten,
- Ermitteln einer Flussgeschwindigkeit des erfassten injizierten Kontrastmittelbolus,
- Festlegen zumindest eines Messparameters der angiographischen Messung gemäß der ermittelten Flussgeschwindigkeit und
- Durchführung der angiographischen Messung des Hauptmessbereichs des Patienten mit dem festgelegten zumindest einen Messparameter in der Magnetresonanzanlage.

Das erfindungsgemäße Verfahren kann derart eine flexible und schnellere Durchführung der angiographischen Messung erlauben. Die Erfindung bietet daher insbesondere mehrere Vorteile:
1) Der zumindest eine Messparameter der angiographischen Messung kann gemäß der ermittelten Flussgeschwindigkeit derart festgelegt werden, dass die angiographische Messung während einer hohen Kontrastierung durch den injizierten Kontrastmittelbolus durchgeführt wird. Für das Festlegen des zumindest einen Messparameters gemäß der patientenindividuell ermittelten Flussgeschwindigkeit des Kontrastmittels und für die Durchführung der angiographischen Messung ist im vorgeschlagenen Vorgehen typischerweise nur eine einmalige Injektion des Kontrastmittelbolus nötig. Vorzugweise kann daher die angiographische Messung sowohl optimal parametrisiert sein als auch rechtzeitig gestartet werden.
2) Der zumindest eine Messparameter der angiographischen Messung wird patientenindividuell, insbesondere bezogen auf den Kreislauf und/oder die Flussgeschwindigkeit des injizierten Kontrastmittelbolus festgelegt. Die während der Durchführung der angiographischen Messung erfassten Messdaten können für eine Rekonstruktion oder eine Erstellung von angiographischen Bildern verwendet werden. Durch die angiographischen Bilder ist vorzugweise eine zuverlässige Befundung bei unterschiedlichen Patienten gewährleistet.
3) Aufgrund der Standardisierung der angiographischen Messung kann auch die Vorbereitung und Durchführung der angiographischen Messung automatisiert werden, beispielsweise durch Landmarkenerkennung.

Der Hauptmessbereich umfasst typischerweise einen Körperbereich des Patienten und insbesondere ein Volumen. Vorzugweise ist der Hauptmessbereich ein Teilbereich eines maximalen Gesichtsfelds der Magnetresonanzanlage. Die angiographische Messung wird üblicherweise in dem Hauptmessbereich des Patienten durchgeführt, so dass während der angiographischen Messung Messdaten erfasst werden können, wobei die von den Messdaten rekonstruierten angiographischen Bilder den Hauptmessbereich abbilden.

Der Patient kann beispielsweise einen Befund oder eine Pathologie aufweisen; alternativ kann der Patient auch einer gesunden Untersuchungsperson entsprechen. Grundsätzlich ist es auch denkbar, dass ein anderes insbesondere tierisches Objekt dem Patienten entsprechen kann.

Die zumindest eine Übersichtsmessung kann einen Übersichtsmessbereich aufweisen, in welchem die Übersichtsmessung durchgeführt wird. Der Übersichtsmessbereich entspricht insbesondere nicht dem Hauptmessbereich des Patienten. Üblicherweise ist der Übersichtsmessbereich größer als der Hauptmessbereich. Der Hauptmessbereich kann typischerweise im Übersichtsmessbereich zumindest teilweise oder insbesondere ganz enthalten sein. Die Übersichtsmessung kann bevorzugt eine kürzere Messzeitdauer als die angiographische Messung aufweisen. Üblicherweise werden vor der Übersichtsmessung patientenspezifische Parameter wie Körpergröße und Körpergewicht, beispielsweise durch einen Benutzer der Magnetresonanzanlage, erfasst. Die Magnetresonanzanlage kann dafür geeignete Mittel aufweisen. Der Benutzer kann insbesondere mit einem Monitor, auf dem eine graphische Benutzeroberfläche angezeigt werden kann, interagieren. Beispielsweise kann die Übersichtsmessung oder insbesondere der Übersichtsmessbereich der Übersichtsmessung durch den Benutzer festgelegt werden. Alternativ oder zusätzlich ist es auch denkbar, dass die zumindest eine Übersichtsmessung automatisch ohne Festlegen des Übersichtsmessbereichs durch den Benutzer durchgeführt wird. Die zumindest eine Übersichtsmessung kann normalerweise Localizer genannt werden. Je nach Konfiguration der zumindest einen Übersichtsmessung können mittels der zumindest einen Übersichtsmessung verschiedene Übersichtsmessdaten generiert werden. Von den verschiedenen Übersichtsmessdaten kann zumindest ein Übersichtsbild rekonstruiert oder erstellt werden. Eine erste Konfiguration der zumindest einen Übersichtsmessung kann beispielsweise dafür geeignet sein, eine Morphologie des Patienten zu erfassen. Eine zweite Konfiguration der zumindest einen Übersichtsmessung ist vorzugweise dafür eingestellt, eine Gefäßstruktur des Patienten (Gefäß-Localizer) zu erfassen.

Grundsätzlich ist es auch denkbar, dass mehr als zumindest eine Übersichtsmessung, beispielsweise zwei Übersichtsmessungen durchgeführt werden. Die erste der zwei Übersichtsmessungen kann beispielsweise derart konfiguriert sein, dass bevorzugt die Morphologie des Patienten in dem Hauptmessbereich erfasst wird. Beispielsweise kann die zweite der zwei Übersichtsmessungen derart konfiguriert sein, dass vorzugweise die Gefäßstruktur des Patienten erfasst wird.

Das Festlegen des Hauptmessbereichs und des ersten Messbereichs kann insbesondere durch den Benutzer geschehen. Beispielsweise kann der Benutzer per Drag'n'Drop den Hauptmessbereich auf dem Monitor festlegen, indem er mit einem Eingabegerät, insbesondere einer Maus, mit der graphischen Benutzeroberfläche auf dem Monitor interagiert. Vorzugweise wird das Festlegen des Hauptmessbereichs und des ersten Messbereichs durch das zumindest eine Übersichtsbild unterstützt. Beispielsweise kann das zumindest eine Übersichtsbild im Hintergrund angezeigt werden und der Benutzer kann den Hauptmessbereich und/oder den ersten Messbereich auf dem zumindest einen Übersichtsbild festlegen. Grundsätzlich ist es auch denkbar, dass der Hauptmessbereich und/oder der erste Messbereich semi-automatisch oder automatisch festgelegt werden. Beispielsweise kann beim semi-automatischen Festlegen der Benutzer aus einer Liste eine Körperregion, beispielsweise Abdomen oder Brust oder Oberschenkel, auf der graphischen Benutzeroberfläche auswählen. Gemäß der Auswahl wird dann insbesondere der Hauptmessbereich dementsprechend festgelegt. Beispielweise erfolgt das semi-automatische oder automatische Festlegen durch Erkennung von Landmarken, welche insbesondere sensorgestützt ist und beispielsweise durch eine Kamera ermöglicht wird. Bei dem automatischen Festlegen des Hauptmessbereichs ist es insbesondere denkbar, dass der Hauptmessbereich ohne Interaktion des Benutzers mit der graphischen Benutzeroberfläche festgelegt wird, beispielsweise basierend auf in einer elektronischen Patientenakte des Patienten enthaltenen Informationen. Analog zum semi-automatischen und automatischen Festlegen des Hauptmessbereichs kann auch das Festlegen des ersten Messbereichs semi-automatisch oder automatisch erfolgen.

Gemäß der Erfindung unterscheidet sich der erste Messbereich vom Hauptmessbereich. Der erste Bereich kann geeignet gemäß dem Hauptmessbereich festgelegt werden. Alternativ kann der Hauptmessbereich gemäß dem ersten Bereich festgelegt sein. Vorzugweise weisen der Hauptmessbereich und der erste Messbereich je einen Teil einer Gefäßstruktur des Patienten auf. Der Hauptmessbereich kann sich vom ersten Messbereich zumindest in einem der folgenden Parameter unterscheiden:
- in einer Ausdehnung des jeweiligen Volumens,
- in einer Orientierung des jeweiligen Volumens und
- in einer Position des jeweiligen Volumens relativ zu einem Koordinatensystem des Patienten.

Vorzugsweise unterscheiden sich der Hauptmessbereich und der erste Messbereich derart, dass das Volumen, welches durch den ersten Messbereich abgedeckt wird, nicht in dem Hauptmessbereich-Volumen enthalten ist und umgekehrt. Beispielsweise weist das Volumen des Hauptmessbereichs die Körperregion Abdomen auf, während der erste Messbereich, insbesondere das Volumen des ersten Messbereichs, die Körperregion Unterarm aufweist. Wenn beispielsweise der Hauptmessbereich die Körperregion Thorax aufweist, kann der erste Messbereich die Körperregion Oberarm aufweisen. In diesem Fall weist normalerweise das maximale Gesichtsfeld die Körperregion Thorax und die Körperregion Oberarm auf.

Üblicherweise weist das maximale Gesichtsfeld der Magnetresonanzanlage den Hauptmessbereich und den ersten Messbereich auf. Falls das maximale Gesichtsfeld den Hauptmessbereich und den ersten Messbereich nicht aufweist, kann beispielsweise der erste Messbereich derart verändert werden, dass das maximale Gesichtsfeld zusätzlich zum Hauptmessbereich den ersten Messbereich aufweist. Vorzugweise kann in diesem Fall lediglich der erste Messbereich verändert werden, während insbesondere der Hauptmessbereich konstant gehalten wird. Beispielsweise kann eine Patientenliege, auf welcher der Patient gelagert ist, derart verfahren werden, dass das maximale Gesichtsfeld den Hauptmessbereich aufweist, oder der Patient wird umgelagert.

Vorzugsweise ist ein Injektionsbereich für eine Injektion des Kontrastmittelbolus abhängig vom festgelegten ersten Messbereich gewählt. Der Injektionsbereich kann derart gewählt sein, dass der Injektionsbereich außerhalb vom maximalen Gesichtsfeld der Magnetresonanzanlage liegt. In diesem Fall kann das maximale Gesichtsfeld den Hauptmessbereich und den ersten Messbereich aufweisen. Beispielsweise kann der Kontrastmittelbolus in den Unterarm des Patienten injiziert werden und der erste Messbereich die Körperregion Oberarm aufweisen. Der Zeitpunkt der Injektion des Kontrastmittelbolus ist vorzugweise mit einem Messstartzeitpunkt der ersten zeitaufgelösten Messung abgestimmt, damit der injizierte Kontrastmittelbolus im ersten Messbereich erfasst werden kann. Vorzugweise liegt der Messstartzeitpunkt der ersten zeitaufgelösten Messung zeitlich vor der Injektion des Kontrastmittelbolus.

Die erste zeitaufgelöste Messung wird mittels der Magnetresonanzanlage in dem festgelegten ersten Messbereich durchgeführt. Derart bilden insbesondere die in der ersten zeitaufgelösten Messung erfassten ersten zeitaufgelösten Messdaten den ersten Messbereich ab. Die erste zeitaufgelöste Messung ist vorzugsweise derart konfiguriert, dass eine Serie von Messdaten, insbesondere erste zeitaufgelöste Messdaten, in dem festgelegten ersten Messbereich über die Zeit generiert werden können. Üblicherweise ist zumindest ein erster Teil der ersten zeitaufgelösten Messdaten zu einem früheren Zeitpunkt erfasst worden als ein zweiter Teil der ersten zeitaufgelösten Messdaten. Vorzugsweise können durch den ersten Teil der ersten zeitaufgelösten Messdaten ein erstes Bild und durch den zweiten Teil der ersten zeitaufgelösten Messdaten ein zweites Bild in dem festgelegten ersten Messbereich werden, wobei das erste Bild den ersten Messbereich zu einem früheren Zeitpunkt abbildet als das zweite Bild. Es können auch mehr Bilder erfasst werden, wenn die erste zeitaufgelöste Messung dementsprechend konfiguriert ist.

Der injizierte Kontrastmittelbolus wird in dem festgelegten ersten Messbereich mittels der ersten zeitaufgelösten Messdaten erfasst. Die erste zeitaufgelöste Messung ist insbesondere derart konfiguriert, dass in den ersten zeitaufgelösten Messdaten der injizierte Kontrastmittelbolus erfasst werden kann. Üblicherweise ist eine Zeitauflösung der ersten zeitaufgelösten Messung zur Erfassung des injizierten Kontrastmittelbolus geeignet festgelegt. Das Erfassen des injizierten Kontrastmittelbolus kann bedeuten, dass lediglich eine unterschiedliche Kontrastierung der ersten zeitaufgelösten Messdaten auf Grund des injizierten Kontrastmittelbolus erfasst wird. Die unterschiedliche Kontrastierung kann durch einen Schwellwert definiert sein. Die erste zeitaufgelöste Messung ist vorzugweise zum Erfassen eines Kontrastierungszeitpunkts geeignet konfiguriert, an welchem sich die Kontrastierung durch den injizierten Kontrastmittelbolus über den Schwellwert hinaus, insbesondere im Vergleich zu einem anderen Zeitpunkt vor der Kontrastierung durch den injizierten Kontrastmittelbolus, unterscheidet.

Die Flussgeschwindigkeit des erfassten injizierten Kontrastmittelbolus wird ermittelt. Die Flussgeschwindigkeit kann ein Maß für eine Blutflussgeschwindigkeit und/oder für eine Kreislaufzeit sein. Von der Flussgeschwindigkeit kann vorzugweise ein allgemeiner Gesundheitszustand des Patienten abgeleitet werden. Die Flussgeschwindigkeit beeinflusst insbesondere einen Kontrastierungszeitraum sowie den Beginn und das Ende einer Kontrastierung in einem bestimmten Messbereich. Der erfasste Kontrastmittelbolus geht in die Ermittlung der Flussgeschwindigkeit ein.

In einer weiteren Ausführung der Erfindung umfasst das Ermitteln der Flussgeschwindigkeit des injizierten Kontrastmittelbolus das Festlegen einer ersten Strecke in dem ersten Messbereich, das Ermitteln einer ersten Laufzeit des im ersten Messbereich erfassten injizierten Kontrastmittelbolus auf der ersten Strecke und das Berechnen der Flussgeschwindigkeit unter Verwendung der ersten Strecke und der ersten Laufzeit. Die erste Strecke kann beispielsweise derart festgelegt werden, dass ein erster Punkt und ein zweiter Punkt in dem ersten Messbereich festgelegt werden. Insbesondere ist es denkbar, dass der Benutzer den ersten Punkt und/oder den zweiten Punkt auf der graphischen Benutzeroberfläche, insbesondere durch das Eingabegerät, festlegt. Vorzugsweise legt der Benutzer auf der graphischen Benutzeroberfläche den ersten Punkt und/oder den zweiten Punkt unter Berücksichtigung der Übersichtsmessdaten fest. Alternativ oder zusätzlich ist es auch denkbar, dass der erste Punkt und der zweite Punkt oder die erste Strecke automatisch festgelegt werden. Beispielsweise weist die Gefäßstruktur des Patienten die erste Strecke auf.

Gemäß einer weiteren Ausführung wird die erste Strecke entlang der mittels der Übersichtsmessdaten erfassten Gefäßstruktur des Patienten festgelegt. Beispielsweise kann dann die erste Strecke durch den Abstand, insbesondere entlang der Gefäßstruktur zwischen dem ersten Punkt und dem zweiten Punkt festgelegt sein. Der erste Punkt und der zweite Punkt können insbesondere je einem ersten Ende und einem zweiten Ende der ersten Strecke entsprechen.

Die erste Laufzeit des im ersten Messbereich erfassten injizierten Kontrastmittelbolus auf der ersten Strecke wird beispielsweise derart ermittelt, dass ein erster Kontrastierungszeitpunkt und ein zweiter Kontrastierungszeitpunkt insbesondere mittels der ersten zeitaufgelösten Messdaten erfasst werden. Beispielsweise werden der erste Kontrastierungszeitpunkt am ersten Punkt im ersten Messbereich und der zweite Kontrastierungszeitpunkt am zweiten Punkt im ersten Messbereich erfasst. Alternativ oder zusätzlich kann der erste Kontrastierungszeitpunkt an dem ersten Ende der ersten Strecke und der zweite Kontrastierungszeitpunkt an dem zweiten Ende der ersten Strecke erfasst werden. Die erste Laufzeit entspricht dann insbesondere dem Betrag der Differenz aus dem ersten Kontrastierungszeitpunkt und dem zweiten Kontrastierungszeitpunkt. Das Ermitteln der ersten Laufzeit kann auch derart erfolgen, dass der Benutzer auf der graphischen Benutzeroberfläche die erste Strecke beispielsweise durch ein Markieren eines Teils der Gefäßstruktur definiert oder die erste Strecke automatisch festgelegt wird und gemäß dem ersten Ende und dem zweiten Ende der ersten Strecke die erste Laufzeit mittels der ersten zeitaufgelösten Messdaten ermittelt wird. Beispielsweise kann die Magnetresonanzanlage zumindest eine Komponente aufweisen, welche basierend auf der ersten Strecke die erste Laufzeit ermitteln kann.

Die Flussgeschwindigkeit wird normalerweise unter Verwendung der ersten Strecke und der ersten Laufzeit berechnet. Beispielsweise wird dafür die erste Strecke durch die erste Laufzeit geteilt.

Der zumindest eine Messparameter der angiographischen Messung wird gemäß der ermittelten Flussgeschwindigkeit festgelegt.

Die angiographische Messung kann mehrere Messparameter aufweisen, welche unterschiedlich klassifiziert werden können. Beispielsweise kann der Messstartzeitpunkt und der Messendzeitpunkt einer ersten Klasse Zeitparameter zugeordnet werden. Eine Matrixgröße, eine räumliche Auflösung, eine Schichtdicke und eine Repetitionszeit werden insbesondere einer zweiten Klasse Sequenzparameter werden. Beispielsweise kann das Verändern des zumindest einen Sequenzparameters eine Anpassung zumindest eines Zeitparameters auslösen und umgekehrt. Der gemäß der ermittelten Flussgeschwindigkeit festgelegte zumindest eine Messparameter wird insbesondere entweder aus der ersten Klasse der Zeitparameter oder der zweiten Klasse der Sequenzparameter gewählt. Derart kann der zumindest eine Messparameter aus der folgenden Liste ausgewählt werden: ein Messstartzeitpunkt, ein Messendzeitpunkt, eine Matrixgröße, eine räumliche Auflösung, eine Schichtdicke, eine Repetitionszeit der angiographischen Messung. Es kann auch eine beliebige Kombination der genannten Messparameter festgelegt werden. Selbstverständlich können weitere, dem Fachmann als sinnvoll erscheinende Messparameter festgelegt werden.

Es ist denkbar, dass das Festlegen des zumindest einen Messparameters der angiographischen Messung durch den Benutzer erfolgt. Vorzugsweise wird der zumindest eine Messparameter automatisch gemäß der ermittelten Flussgeschwindigkeit festgelegt. Denn üblicherweise ist eine Verzögerung zwischen dem Erfassen des injizierten Kontrastmittelbolus im ersten Messbereich und der voraussichtlichen Kontrastierung im Hauptmessbereich nicht ausreichend, dass der zumindest eine Messparameter manuell, insbesondere durch den Benutzer, festgelegt werden kann.

Das Festlegen des zumindest einen Messparameters kann zur Folge haben, dass ein anderer Messparameter automatisch verändert wird. Durch das Festlegen des Messstartzeitpunkts der angiographischen Messung kann ein Messendzeitpunkt der ersten zeitaufgelösten Messung festgelegt werden, beispielsweise derart, dass die angiographische Messung nach einer Pause oder unmittelbar nach der ersten zeitaufgelösten Messung gestartet wird. Daraus kann beispielsweise folgen, dass die erste zeitaufgelöste Messung nach dem Erfassen des injizierten Kontrastmittelbolus beendet und die angiographische Messung gestartet wird. In anderen Worten kann durch das Festlegen des zumindest einen Messparameters der angiographischen Messung ein anderer zumindest eine Messparameter einer anderen Messung, beispielsweise der zeitaufgelösten Messung festgelegt sein und umgekehrt.

Der zumindest eine Messparameter kann beispielsweise einem Messstartzeitpunkt oder einem Messendzeitpunkt der angiographischen Messung entsprechen. Der Messstartzeitpunkt der angiographischen Messung und der Messendzeitpunkt der angiographischen Messung können insbesondere eine Messzeitdauer der angiographischen Messung festlegen. Beispielsweise können gleichzeitig der Messstartzeitpunkt und der Messendzeitpunkt der angiographischen Messung gemäß der ermittelten Flussgeschwindigkeit festgelegt werden. Vorzugsweise wird der zumindest eine Messparameter der angiographischen Messung derart festgelegt, dass die angiographische Messung in einem Zeitraum mit, insbesondere optimaler, Kontrastierung durch den injizierten Kontrastmittelbolus in dem Hauptmessbereich durchgeführt wird. Der zumindest eine Messparameter der angiographischen Messung wird normalerweise derart festgelegt, dass besonders geeignete angiographische Bilder während der Durchführung der angiographischen Messung ermittelt werden können.

Der zumindest eine Messparameter kann derart festgelegt sein, dass die angiographische Messung innerhalb der Messzeitdauer durchgeführt wird. Die Sequenzparameter und die Zeitparameter sind üblicherweise für einen Kontrast, eine Signalstärke und die Messzeitdauer der angiographischen Messung verantwortlich. Vorteilhafterweise ist der zumindest eine Messparameter zur Durchführung der angiographischen Messung in Hinblick auf den Kontrast, die Signalstärke und/oder die Messzeitdauer der angiographischen Messung angepasst.

Beispielsweise kann die Messzeitdauer der angiographischen Messung verlängert werden, indem der zeitliche Abstand zwischen dem Messstartzeitpunkt der angiographischen Messung und dem Messendzeitpunkt der angiographischen Messung vergrößert wird. Durch das Festlegen des zumindest einen Messparameters kann insbesondere die Messzeitdauer der angiographischen Messung in Abhängigkeit von der ermittelten Flussgeschwindigkeit festgelegt werden. Beispielsweise ist die Messzeitdauer der angiographischen Messung desto länger, je langsamer die Flussgeschwindigkeit ist. Die Messzeitdauer der angiographischen Messung ist üblicherweise umso länger, je höher die erste Laufzeit ist.

Vorzugweise kann das Festlegen des zumindest einen Messparameters der angiographischen Messung lediglich das Festlegen der Zeitparameter umfassen, insbesondere beispielweise das Verschieben des Messstartzeitpunkts der angiographischen Messung und des Messendzeitpunkts der angiographischen Messung im gleichen Maße. Wenn der Messstartzeitpunkt der angiographischen Messung und der Messendzeitpunkt der angiographischen Messung im gleichen Maße verschoben wird, müssen üblicherweise keine weiteren Sequenzparameter angepasst werden, weil die Messzeitdauer der angiographischen Messung dadurch unverändert sein kann.

Beispielsweise kann bereits vor dem Durchführen der ersten zeitaufgelösten Messung der zumindest eine Messparameter der angiographischen Messung festgelegt sein. Vor dem Ermitteln der Flussgeschwindigkeit des injizierten Kontrastmittelbolus kann insbesondere der zumindest eine Messparameter gemäß der Standardwerte für die angiographische Messung oder basierend auf vergleichbaren angiographischen Messungen bei anderen Patienten festgelegt sein. Nach dem Ermitteln der Flussgeschwindigkeit kann der zumindest eine Messparameter erneut gemäß der Flussgeschwindigkeit festgelegt werden bzw. gemäß der ermittelten Flussgeschwindigkeit angepasst werden.

Dies ist insbesondere vorteilhaft, wenn beispielsweise die Kreislaufzeiten der Patienten sich unterscheiden oder insbesondere Pathologien vorliegen oder andere anatomische Größen unter den Patienten variieren. Die angiographische Messung ist insbesondere patientenindividuell durchführbar.

Die angiographische Messung des Hauptmessbereichs des Patienten wird mit dem festgelegten zumindest einen Messparameter in der Magnetresonanzanlage durchgeführt. Während der Durchführung der angiographischen Messung werden angiographische Messdaten generiert. Von den angiographischen Messdaten können angiographische Bilder rekonstruiert werden, welche insbesondere zur Erstellung einer Angiographie verwendet werden.

In einer weiteren Ausführung wird mittels der Übersichtsmessdaten ein zweiter Messbereich, welcher sich vom ersten Messbereich und vom Hauptmessbereich unterscheidet, festgelegt. Der zweite Messbereich unterscheidet sich bevorzugt vom Hauptmessbereich derart, dass der Hauptmessbereich analog zum ersten Messbereich nicht den zweiten Messbereich aufweist und umgekehrt.

In einer Ausführungsform ist der erste Messbereich relativ zum Hauptmessbereich distal gelegen und der zweite Messbereich relativ zum Hauptmessbereich proximal gelegen. Der erste Messbereich und der zweite Messbereich sind bevorzugt derart festgelegt, dass der injizierte Kontrastmittelbolus zunächst im ersten Messbereich und daraufhin im zweiten Messbereich erfasst werden kann. Üblicherweise ist daher der Abstand zwischen dem zweiten Messbereich und dem Hauptmessbereich geringer als der Abstand zwischen dem ersten Messbereich und dem Hauptmessbereich.

Grundsätzlich ist es gemäß einer weiteren Ausführung denkbar, dass lediglich der erste Messbereich oder lediglich der zweite Messbereich abhängig vom Hauptmessbereich automatisch festgelegt werden. Alternativ oder zusätzlich ist es auch denkbar, dass der erste Messbereich und der zweite Messbereich abhängig vom Hauptmessbereich insbesondere automatisch festgelegt werden. Beispielsweise kann der Hauptmessbereich über eine Vorgabe der zu untersuchenden Körperregion festgelegt sein und dementsprechend der erste Messbereich und/oder der zweite Messbereich automatisch festgelegt werden. Wenn der erste Messbereich und/oder der zweite Messbereich festgelegt sind, werden üblicherweise die jeweilige erste zeitaufgelöste Messung und die zweite zeitaufgelöste Messung automatisch zugeordnet und vorzugweise konfiguriert.

Gemäß einer weiteren Ausführung weist das maximale Gesichtsfeld der Magnetresonanzanlage den ersten Messbereich, den zweiten Messbereich und den Hauptmessbereich auf. In diesem Fall kann die Magnetresonanzanlage eine Messung in dem jeweiligen Messbereich ohne Umlagerung des Patienten oder Tischvorschub der Patientenliege durchführen. Bevorzugt kann der Patient derart in der Magnetresonanzanlage positioniert sein, dass der Hauptmessbereich der angiographischen Messung in dem maximalen Gesichtsfeld enthalten ist. Der erste Messbereich und/oder der zweite Messbereich können insbesondere gemäß dem Hauptmessbereich festlegt werden, vorzugsweise derart, dass der erste Messbereich und/oder der zweite Messbereich ebenfalls im maximalen Gesichtsfeld der Magnetresonanzanlage liegen.

Gemäß einer Ausführungsform umfasst das Festlegen des zumindest einen Messparameters der angiographischen Messung das Durchführen einer zweiten zeitaufgelösten Messung mittels der Magnetresonanzanlage in dem festgelegten zweiten Messbereich, wobei zweite zeitaufgelöste Messdaten generiert werden, das Erfassen des injizierten Kontrastmittelbolus in dem zweiten Messbereich mittels der zweiten zeitaufgelösten Messdaten und das Festlegen des Messstartzeitpunkts der angiographischen Messung abhängig von dem in dem zweiten Messbereich erfassten injizierten Kontrastmittelbolus.

Die zweite zeitaufgelöste Messung ist vorzugsweise derart konfiguriert, dass eine zweite Serie von Messdaten, insbesondere zweite zeitaufgelöste Messdaten, in dem festgelegten zweiten Messbereich über die Zeit generiert werden können. Üblicherweise ist zumindest ein erster Teil der zweiten zeitaufgelösten Messdaten zu einem früheren Zeitpunkt erfasst worden als ein zweiter Teil der zweiten zeitaufgelösten Messdaten. Vorzugsweise können durch den ersten Teil der zweiten zeitaufgelösten Messdaten ein erstes Bild und durch den zweiten Teil der zweiten zeitaufgelösten Messdaten ein zweites Bild in dem festgelegten zweiten Messbereich werden, wobei das erste Bild den zweiten Messbereich zu einem früheren Zeitpunkt abbildet als das zweite Bild. Es können auch mehr Bilder erfasst werden, wenn die erste zeitaufgelöste Messung dementsprechend konfiguriert ist.

Des Weiteren kann der injizierte Kontrastmittelbolus insbesondere in dem festgelegten zweiten Messbereich mittels der zweiten zeitaufgelösten Messdaten erfasst werden. Die zweite zeitaufgelöste Messung kann vorzugsweise derart konfiguriert sein, dass die zweiten zeitaufgelösten Messdaten den injizierten Kontrastmittelbolus aufweisen können. Üblicherweise ist eine Zeitauflösung der zweiten zeitaufgelösten Messung derart festgelegt, dass der injizierte Kontrastmittelbolus mittels der zweiten zeitaufgelösten Messdaten erfasst werden kann. Das Erfassen des injizierten Kontrastmittelbolus kann bedeuten, dass lediglich eine unterschiedliche Kontrastierung der zweiten zeitaufgelösten Messdaten auf Grund des injizierten Kontrastmittelbolus erfasst wird. Die unterschiedliche Kontrastierung kann durch einen Schwellwert definiert sein. Die zweite zeitaufgelöste Messung ist vorzugweise zum Erfassen eines dritten Kontrastierungszeitpunkts geeignet konfiguriert, an welchem sich die Kontrastierung durch den injizierten Kontrastmittelbolus über den Schwellwert hinaus insbesondere im Vergleich zu einem anderen Zeitpunkt vor der Kontrastierung durch den injizierten Kontrastmittelbolus unterscheidet.

Der Messstartzeitpunkt der angiographischen Messung kann insbesondere gemäß dem dritten Kontrastierungszeitpunkt festgelegt werden. Das Erfassen des injizierten Kontrastmittelbolus im zweiten Messbereich kann dem Ermitteln des dritten Kontrastierungszeitpunkts entsprechen. Üblicherweise kann, nachdem der dritte Kontrastierungszeitpunkt im zweiten Messbereich mittels der zweiten zeitaufgelösten Messung erfasst worden ist, der Messstartzeitpunkt der angiographischen Messung festgelegt werden. Üblicherweise wird der Messstartzeitpunkt der angiographischen Messung unmittelbar nach dem Erfassen des dritten Kontrastierungszeitpunkts festgelegt. Vorzugweise kann der Messstartzeitpunkt der angiographischen Messung derart festgelegt sein, dass die angiographische Messung unmittelbar nach der zweiten zeitaufgelösten Messung durchgeführt wird. Durch das Festlegen des Messstartzeitpunkts der angiographischen Messung kann auch ein Messendzeitpunkt der zweiten zeitaufgelösten Messung festgelegt werden. Beispielsweise wird die zweite zeitaufgelöste Messung unmittelbar nach dem Erfassen des injizierten Kontrastmittelbolus beendet mittels des Festlegens des Messendzeitpunkts der zweiten zeitaufgelösten Messung. Weiterhin kann in diesem Fall die angiographische Messung nach einer Pause oder unmittelbar gestartet werden, wenn der Messstartzeitpunkt der angiographischen Messung entsprechend festgelegt wird.

Der injizierte Kontrastmittelbolus kann vorzugweise sowohl im ersten Messbereich als auch im zweiten Messbereich sowie im Hauptmessbereich erfasst werden. Das bedeutet insbesondere, dass für die Durchführung der angiographischen Messung lediglich die Injektion eines einzigen Kontrastmittelbolus nötig ist. Die erste zeitaufgelöste Messung, die zweite zeitaufgelöste Messung und die angiographische Messung sowie der Zeitpunkt der Injektion des Kontrastmittelbolus können zusammen ein angiographisches Messprotokoll angeben. Bei einer ungeeigneten Konfiguration des angiographischen Messprotokolls, beispielsweise durch eine fehlerhafte zeitliche Reihenfolge oder ungeeignete Konfiguration der ersten zeitaufgelösten Messung, der zweiten zeitaufgelösten Messung, der angiographischen Messung und dem Zeitpunkt der Injektion des Kontrastmittelbolus kann passieren, dass der injizierte Kontrastmittelbolus nicht erfasst werden kann. Die erste zeitaufgelöste Messung, die zweite zeitaufgelöste Messung und die angiographische Messung sind üblicherweise nacheinander durchzuführen, wobei insbesondere die erste zeitaufgelöste Messung zeitlich vor der zweiten zeitaufgelösten Messung durchgeführt wird und die zweite zeitaufgelöste Messung zeitlich vor der angiographische Messung.

Wenn das angiographische Messprotokoll die zweite zeitaufgelöste Messung aufweist, kann üblicherweise nach dem Erfassen des injizierten Kontrastmittelbolus im ersten Messbereich die erste zeitaufgelöste Messung beendet und die zweite zeitaufgelöste Messung gestartet werden. Nach dem Erfassen des injizierten Kontrastmittelbolus im zweiten Messbereich kann insbesondere die zweite zeitaufgelöste Messung beendet und die angiographische Messung gestartet werden.

Wenn der injizierte Kontrastmittelbolus mittels der zweiten zeitaufgelösten Messung erfasst wird, kann der zumindest eine Messparameter bevorzugt derart festgelegt sein, dass die Differenz zwischen dem Messstartzeitpunkt der angiographischen Messung und dem Messendzeitpunkt der angiographischen Messung sowie insbesondere die Messzeitdauer der angiographischen Messung konstant gehalten werden. Alternativ oder zusätzlich ist es auch denkbar, dass mittels des Festlegens des Messstartzeitpunkts der angiographischen Messung abhängig von dem in dem zweiten Messbereich erfassten injizierten Kontrastmittelbolus beispielsweise zumindest ein Zeitparameter und/oder zumindest ein Sequenzparameter festgelegt oder angepasst wird. Wenn zumindest ein Sequenzparameter festlegt wird, beispielsweise derart, dass die Messzeitdauer der angiographischen Messung variiert, kann ein erneutes Festlegen eines weiteren zumindest einen Sequenzparameter erfolgen.

Der zumindest eine Messparameter der angiographischen Messung wird insbesondere unter Verwendung einer zweiten Laufzeit des injizierten Kontrastmittelbolus festgelegt, wobei eine zweite Strecke zwischen dem zweiten Messbereich und dem Hauptmessbereich festgelegt wird und die zweite Laufzeit des injizierten Kontrastmittelbolus entlang der zweiten Strecke mittels der in dem ersten Messbereich ermittelten Flussgeschwindigkeit des injizierten Kontrastmittelbolus berechnet wird. Die zweite Laufzeit des injizierten Kontrastmittelbolus kann dem Betrag der Differenz zwischen einem vierten Kontrastierungszeitpunkt und einem fünften Kontrastierungszeitpunkt entsprechen. Beispielsweise kann durch das jeweilige Erfassen des injizierten Kontrastmittelbolus an einem ersten Ende der zweiten Strecke und an einem zweiten Ende der zweiten Strecke der vierte Kontrastierungszeitpunkt und der fünfte Kontrastierungszeitpunkt ermittelt werden. Alternativ oder zusätzlich kann der vierte Kontrastierungszeitpunkt und der fünfte Kontrastierungszeitpunkt derart ermittelt werden, in dem die zweite Strecke festgelegt wird. Die zweite Laufzeit kann entweder durch den Benutzer der Magnetresonanzanlagen oder insbesondere automatisch ermittelt werden.

Die zweite Strecke wird bevorzugt von dem Benutzer der Magnetresonanzanlage beispielsweise auf der graphischen Benutzeroberfläche festgelegt. Dafür kann der Benutzer mit der graphischen Benutzeroberfläche interagieren. Beispielsweise wird das Festlegen der zweiten Strecke durch die Übersichtsmessdaten unterstützt. Die zweite Strecke befindet sich üblicherweise zwischen dem zweiten Messbereich und dem Hauptmessbereich. Vorzugweise können der zweite Messbereich und der Hauptmessbereich je entweder das erste Ende der zweiten Strecke oder das zweite Ende der zweiten Strecke aufweisen. In einer weiteren Ausführung wird die zweite Strecke entlang der mittels der Übersichtsmessdaten erfassten Gefäßstruktur des Patienten festgelegt.

Der zumindest eine Messparameter der angiographischen Messung kann an die zweite Laufzeit des injizierten Kontrastmittelbolus angepasst sein. Beispielsweise kann der Messstartzeitpunkt der angiographischen Messung umso später festgelegt werden, je höher die zweite Laufzeit ist.

In einer weiteren Ausführungsform wird die zweite Strecke zwischen dem zweiten Messbereich und einem Teilbereich des Hauptmessbereichs mittels der Übersichtsmessdaten ermittelt. Der Teilbereich des Hauptmessbereichs kann üblicherweise vollständig im Hauptmessbereich enthalten sein. Üblicherweise ist der Teilbereich kleiner als der Hauptmessbereich. Vorzugweise kann der Teilbereich des Hauptmessbereichs eine für medizinisch relevante Struktur oder Morphologie, insbesondere arterielle und/oder venöse Gefäße der Gefäßstruktur aufweisen.

In einer Ausführung umfasst das Festlegen des zumindest einen Messparameters der angiographischen Messung unter Verwendung der zweiten Laufzeit des injizierten Kontrastmittelbolus das Festlegen eines Mittelzeitpunkts der angiographischen Messung, welcher einem Erfassen eines Zentralbereichs eines k-Raums des Hauptmessbereichs entspricht, abhängig von der zweiten Laufzeit. Der Mittelzeitpunkt entspricht insbesondere dem Zeitpunkt, bei dem die angiographische Messung den Zentralbereich des k-Raums erfasst. Der Zentralbereich weist üblicherweise einen Bereich um einen Null-Punkt des k-Raums auf. Insbesondere kann der Zentralbereich des k-Raums die niederfrequenten Anteile der angiographischen Messung enthalten im Vergleich zu den hochfrequenten Anteilen der angiographischen Messung in einem peripheren Teil des k-Raums. Der Zentralbereich des k-Raums ist daher vorzugsweise für den Kontrast, der periphere Teil des k-Raums eher für die Details der angiographischen Messung verantwortlich. Zwischen dem Messstartzeitpunkt der angiographischen Messung und dem Messendzeitpunkts der angiographischen Messung liegt üblicherweise der Mittelzeitpunkt.

Wenn der Mittelzeitpunkt abhängig von der zweiten Laufzeit festgelegt wird, kann der Zentralbereich eines k-Raums des Hauptmessbereichs insbesondere einem solchen Teilbereich entsprechen, der zumindest das erste Ende oder das zweite Ende der zweiten Strecke aufweist. Vorzugweise kann insbesondere der Teilbereich des Hauptmessbereichs mittels der angiographischen Messung derart erfasst werden, dass der Mittelzeitpunkt einem Zeitpunkt der maximalen Kontrastierung durch den injizierten Kontrastmittelbolus entspricht.

Grundsätzlich ist denkbar, dass die Flussgeschwindigkeit in dem ersten Messbereich ermittelt wird, gemäß welcher der zumindest eine Messparameter festgelegt wird, und dass durch das Erfassen des injizierten Kontrastmittelbolus mittels der ersten zeitaufgelösten Messung ebenso der Messstartzeitpunkt der angiographischen Messung festgelegt wird. Diese Konfiguration ist besonders in diesen Fällen möglich, bei der der erste Messbereich so weit von dem Hauptmessbereich entfernt ist, dass das Festlegen des zumindest einen Messparameters insbesondere vor der Kontrastierung des Hauptmessbereichs durch den injizierten Kontrastmittelbolus technisch realisierbar ist und die angiographische Messung mit dem festgelegten zumindest einen Messparameter gestartet werden kann. Wenn der erste Messbereich zu weit von dem Hauptmessbereich festgelegt ist oder eine dritte Strecke zwischen dem ersten Messbereich und dem Hauptmessbereich nicht ermittelt werden kann, kann es insbesondere unvorteilhaft sein, wenn die angiographische Messung nach einer Pause oder unmittelbar nach dem Erfassen des injizierten Kontrastmittelbolus in dem ersten Messbereich gestartet wird.

Vorteilhafterweise werden die erste zeitaufgelöste Messung, die zweite zeitaufgelöste Messung und die angiographische Messung automatisch durchgeführt, wobei der jeweilige zugehörige erste Messbereich, zweite Messbereich oder Hauptmessbereich im maximalen Gesichtsfeld der Anlage positioniert sind. Beispielsweise kann die erste zeitaufgelöste Messung, die zweite zeitaufgelöste Messung und die angiographische Messung mehrmals, vorzugweise ohne Anpassung der Messparameter durchgeführt werden. Üblicherweise wird lediglich die angiographische Messung zweimal durchgeführt. Insbesondere wird nach einer ersten Durchführung der angiographischen Messung und vor der zweiten Durchführung der angiographischen Messung der Kontrastmittelbolus injiziert. Beispielsweise kann die Injektion mittels eines Injektors durchgeführt werden. Der Zeitpunkt der Injektion kann typischerweise durch den Benutzer oder automatisiert festgelegt sein. Beispielsweise wird der Hauptmessbereich daher zweimal mit der angiographischen Messung gemessen, wobei sich die Messparameter der beiden angiographischen Messungen vorzugsweise nicht und lediglich sich die Zeitparameter, insbesondere der Messstartzeitpunkt und der Messendzeitpunkt der beiden angiographischen Messungen unterscheiden.

Typischerweise kann die Angiographie anhand der durchgeführten angiographischen Messungen erstellt werden. Vorzugweise werden angiographische Bilder basierend auf den angiographischen Messungen ermittelt, um mittels der angiographischen Bilder insbesondere die Angiographie zu erstellen. Üblicherweise wird die Angiographie des Patienten durch die Subtraktion der angiographischen Bilder vor der Injektion des Kontrastmittels und der angiographischen Bilder nach der Injektion des Kontrastmittels erstellt. Die Angiographie entspricht in diesem Fall im Wesentlichen lediglich einem Erfassen des injizierten Kontrastmittelbolus innerhalb der Gefäßstruktur des Patienten. Vorzugweise wird die Angiographie basierend auf den angiographischen Bildern des Hauptmessbereichs automatisch mittels geeigneter Algorithmen erstellt. Alternativ oder zusätzlich kann auch der Benutzer die angiographischen Bilder auf der graphischen Benutzeroberfläche separat betrachten oder manuell zu der Angiographie des Patienten zusammenfügen.

Neben dem Verfahren betrifft die Erfindung auch eine Magnetresonanzanlage, umfassend eine Planungseinheit, eine Steuereinheit und eine Messeinheit, die zur Durchführung des erfindungsgemäßen Verfahrens ausgebildet ist. Die oben getätigten Ausführungen zum erfindungsgemäßen Verfahren lassen sich analog auf die Magnetresonanzanlage übertragen. Die Magnetresonanzanlage ist somit dazu ausgelegt ein Verfahren zur Durchführung einer angiographischen Messung von einem Hauptmessbereich eines Patienten auszuführen.

Die Magnetresonanzanlage kann weitere Komponenten aufweisen, welche zum Ausführen eines erfindungsgemäßen Verfahrens nötig und/oder vorteilhaft sind, insbesondere eine Kontrastmittelinjektionsvorrichtung. Auf einer Speichereinheit der Planungseinheit und/oder der Steuereinheit und/oder der Messeinheit können Computerprogramme und weitere Software gespeichert sein, mittels derer ein Prozessor der Planungseinheit und/oder der Steuereinheit und/oder der Messeinheit einen Verfahrensablauf eines erfindungsgemäßen Verfahrens automatisch steuert und/oder ausführt.

Die Komponenten der erfindungsgemäßen Magnetresonanzanlage können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das erfindungsgemäße Computerprogrammprodukt ist direkt in einen Speicher einer programmierbaren Steuereinheit einer Magnetresonanzanlage ladbar und weist Programmcode-Mittel auf, um ein erfindungsgemäßes Verfahren auszuführen, wenn das Computerprogrammprodukt in der Steuereinheit der Magnetresonanzanlage ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Steuereinheit die erfindungsgemäßen Verfahrensschritte ausführen kann. Die Steuereinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der Magnetresonanzanlage direkt verbunden oder als Teil der Magnetresonanzanlage ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Steuereinheit der Magnetresonanzanlage ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in die Steuereinheit und/oder in die Planungseinheit und/oder in die Messeinheit der Magnetresonanzanlage gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: zeigt eine erfindungsgemäße Magnetresonanzanlage schematisch,
- Fig. 2: zeigt ein Flussdiagramm eines erfindungsgemäßen Verfahrens zur Durchführung einer angiographischen Messung von einem Hauptmessbereich eines Patienten mittels einer Magnetresonanzanlage,
- Fig. 3: illustriert das Festlegen des Hauptmessbereichs 303, eines ersten Messbereichs und eines zweiten Messbereichs mittels der Übersichtsmessdaten und
- Fig. 4: zeigt das Erfassen des Kontrastmittelbolus in einem kontrastierten Gefäß schematisch.

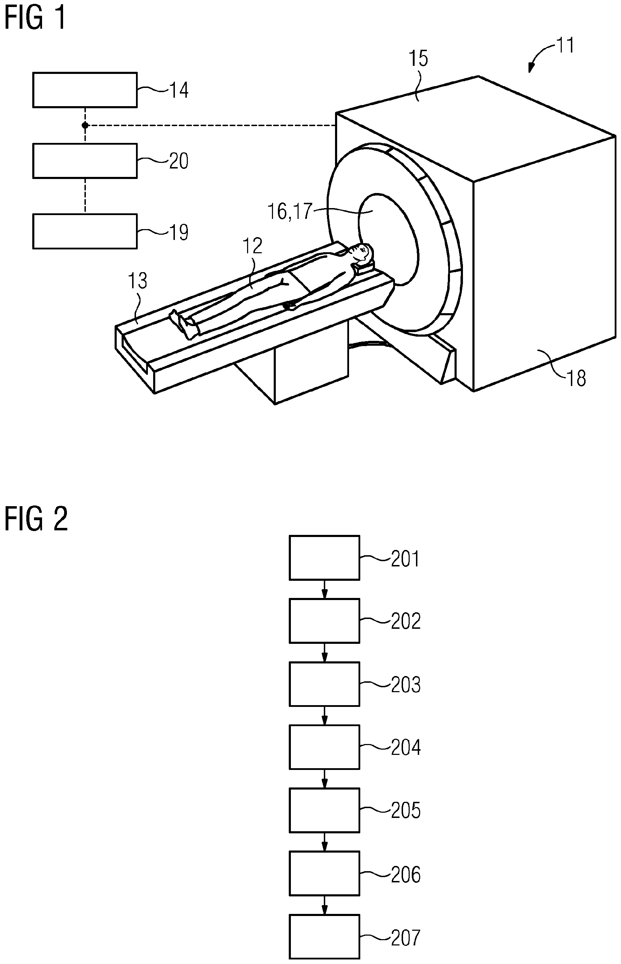

Fig. 1 stellt eine erfindungsgemäße Magnetresonanzanlage 11 schematisch dar. Die Magnetresonanzanlage 11 umfasst eine von einer Magneteinheit 15 gebildeten Detektoreinheit mit einem Hauptmagneten zu einem Erzeugen eines starken und insbesondere konstanten Hauptmagnetfelds. Zudem weist die Magnetresonanzanlage 11 einen zylinderförmigen Patientenaufnahmebereich 16 zu einer Aufnahme eines Patienten 12 auf, wobei der Patientenaufnahmebereich 16 in einer Umfangsrichtung von der Magneteinheit 15 zylinderförmig umschlossen ist.

Im vorliegenden Fall kann der Patient 12 mittels einer Patientenlagerungsvorrichtung 13 der Magnetresonanzanlage 11 in den Patientenaufnahmebereich 16 der Magnetresonanzanlage 11 geschoben werden. Die Patientenlagerungsvorrichtung 13 weist hierzu einen Liegentisch auf, der bewegbar innerhalb der Magnetresonanzanlage 11 angeordnet ist. Der Patientenaufnahmebereich 16 der Magnetresonanzanlage 11 weist ein maximales Gesichtsfeld 17 mit einer Ausdehnung in Längsrichtung der Patientenlagerungsvorrichtung 13 der Magnetresonanzanlage 11 auf.

Die Magneteinheit 15 ist mittels einer Gehäuseverkleidung der Magnetresonanzanlage 11 nach außen abgeschirmt. Die Magneteinheit 13 weist weiterhin eine Gradientenspuleneinheit zu einer Erzeugung von Magnetfeldgradienten auf, die für eine Ortskodierung während einer Bildgebung verwendet werden. Die Gradientenspuleneinheit wird mittels einer Gradientensteuereinheit angesteuert. Des Weiteren weist die Magneteinheit eine Hochfrequenzantenneneinheit, welche im gezeigten Fall als fest in die Magnetresonanzanlage 11 integrierte Körperspule ausgebildet ist, und eine Hochfrequenzantennensteuereinheit zu einer Anregung einer Polarisation, die sich in dem von einem Hauptmagneten der Magneteinheit 15 erzeugten Hauptmagnetfeld einstellt, auf. Die Hochfrequenzantenneneinheit wird von der Hochfrequenzantennensteuereinheit angesteuert und strahlt hochfrequente Magnetresonanz-Sequenzen in einen Untersuchungsraum, der im Wesentlichen von dem Patientenaufnahmebereich 16 gebildet ist, ein. Die Hochfrequenzantenneneinheit ist weiterhin zum Empfang von Magnetresonanz-Signalen, insbesondere aus dem Patienten 12, ausgebildet.

Die Magnetresonanzanlage 11 weist weiterhin eine Messeinheit 18 auf. Zum Beispiel kann die Messeinheit 18 Teil der Magneteinheit 15 sein. Die Messeinheit 18 kann die Gradientenspuleneinheit und/oder die Hochfrequenzantenneneinheit aufweisen.

Zu einer Steuerung der Magneteinheit 15 weist die Magnetresonanzanlage 11 eine Steuereinheit 14 auf. Die Steuereinheit 14 steuert zentral die Magnetresonanzanlage 11, wie beispielsweise das Durchführen einer vorbestimmten bildgebenden Gradientenechosequenz.

Steuerinformationen wie beispielsweise Messparameter, sowie rekonstruierte Magnetresonanz-Bilder können auf einer Anzeigeeinheit 19, beispielsweise auf zumindest einem Monitor, der Magnetresonanzanlage 11 für einen Benutzer angezeigt werden. Zudem können Steuerinformationen zwischen der Steuereinheit 14 und einer Planungseinheit 20 ausgetauscht werden. Typischerweise umfasst die Planungseinheit 20 die Anzeigeeinheit 19 und ein Eingabegerät, beispielsweise eine Maus oder Tastatur. Beispielsweise kann die Steuereinheit 14 Messparameter der Planungseinheit zur Verfügung stellen, damit die Planungseinheit 20 diese Messparameter auf zumindest einem Monitor für den Benutzer angezeigt werden. In einem weiteren Schritt kann die Planungseinheit 20 beispielsweise Messparameter festlegen und diese der Steuereinheit 14 bereitstellen. Mittels der Planungseinheit 20 können Informationen und/oder Messparameter während eines Messvorgangs von einem Benutzer eingegeben werde.

Die dargestellte Magnetresonanzanlage 11 kann selbstverständlich weitere Komponenten umfassen, die Magnetresonanzanlagen 11 gewöhnlich aufweisen, beispielsweise einen Injektor zur Injektion des Kontrastmittels. Ebenso können die einzelnen Komponenten, insbesondere die Anzeigeeinheit 19, die Steuereinheit 14, die Messeinheit 18 und die Planungseinheit 20 in anderer Beziehung zueinander stehen und/oder in eine übergeordnete Einheit integriert sein. Eine allgemeine Funktionsweise einer Magnetresonanzanlage 11 ist zudem dem Fachmann bekannt, so dass auf eine detaillierte Beschreibung der weiteren Komponenten verzichtet wird.

Fig. 2 zeigt ein Flussdiagramm eines erfindungsgemäßen Verfahrens zur Durchführung einer angiographischen Messung von einem Hauptmessbereich 303 eines Patienten 12 mittels einer Magnetresonanzanlage 11. Das Verfahren umfasst die Verfahrensschritte 201 - 207, wobei bei der Beschreibung der Verfahrensschritte 201 - 207, auch Beschreibungsteile einschließlich der entsprechenden im Zusammenhang mit den anderen Figuren eingeführten Bezugszeichen verwendet werden.

Verfahrensschritt 201 kennzeichnet das Durchführen zumindest einer Übersichtsmessung mittels der Magnetresonanzanlage 11, wobei Übersichtsmessdaten generiert werden.

Verfahrensschritt 202 kennzeichnet das Festlegen des Hauptmessbereichs 303 und eines ersten Messbereichs 301, welcher sich vom Hauptmessbereich 303 unterscheidet, mittels der Übersichtsmessdaten.

Verfahrensschritt 203 kennzeichnet das Durchführen einer ersten zeitaufgelösten Messung mittels der Magnetresonanzanlage 11 in dem festgelegten ersten Messbereich 303, wobei erste zeitaufgelöste Messdaten generiert werden.

Verfahrensschritt 204 kennzeichnet das Erfassen eines injizierten Kontrastmittelbolus in dem festgelegten ersten Messbereich 301 mittels der ersten zeitaufgelösten Messdaten.

Verfahrensschritt 205 kennzeichnet das Ermitteln einer Flussgeschwindigkeit 311.F des erfassten injizierten Kontrastmittelbolus.

Verfahrensschritt 206 kennzeichnet das Festlegen zumindest eines Messparameters der angiographischen Messung gemäß der ermittelten Flussgeschwindigkeit 311.F und insbesondere das Festlegen einer Messzeitdauer der angiographischen Messung in Abhängigkeit von der ermittelten Flussgeschwindigkeit 311.F.

Verfahrensschritt 207 kennzeichnet die Durchführung der angiographischen Messung des Hauptmessbereichs 303 des Patienten 12 mit dem festgelegten zumindest einen Messparameter in der Magnetresonanzanlage 11.

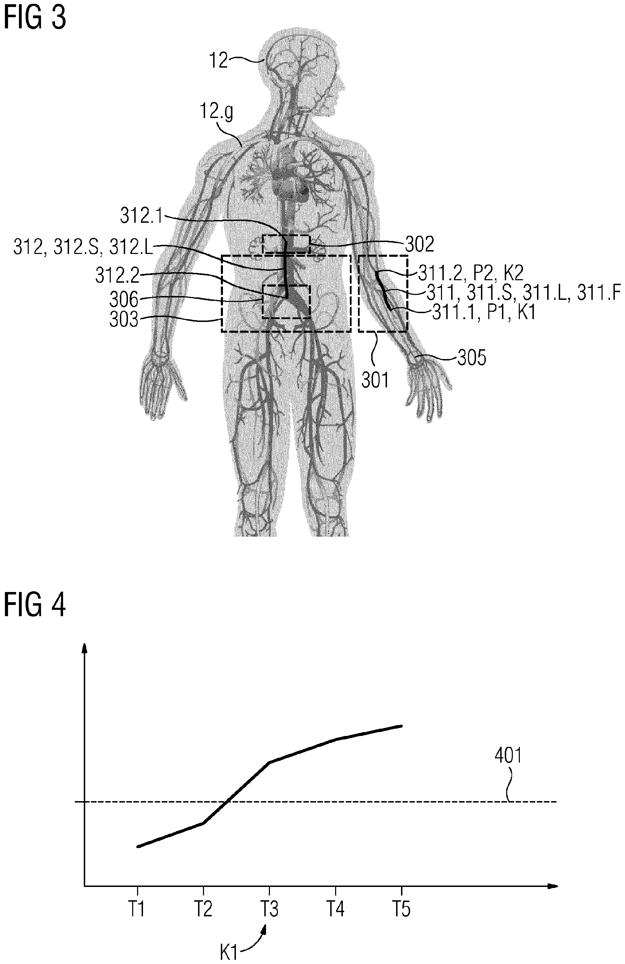

Fig. 3 illustriert das Festlegen des Hauptmessbereichs 303, eines ersten Messbereichs 301 und eines zweiten Messbereichs 302 mittels der Übersichtsmessdaten. Der erste Messbereich 301, der zweite Messbereich 302 und der Hauptmessbereich 303 unterscheiden sich jeweils voneinander. Das maximale Gesichtsfeld 17 weist den ersten Messbereich 301, den zweiten Messbereich 302 und den Hauptmessbereich 303 auf. In dieser Ausgestaltung ist der erste Messbereich 301 relativ zum Hauptmessbereich 303 distal und der zweite Messbereich 302 relativ zum Hauptmessbereich 303 proximal gelegen. Der erste Messbereich 301 und/oder der zweite Messbereich 302 können abhängig vom Hauptmessbereich 303 automatisch festgelegt werden.

Der Kontrastmittelbolus kann vorzugweise an einer Injektionsstelle 305 injiziert werden, wobei weder der erste Messbereich 301, der zweite Messbereich 302 noch der Hauptmessbereich 303 die Injektionsstelle 305 aufweisen. Vorzugsweise ist die Injektionsstelle 305 derart gewählt, dass zunächst der erste Messbereich 301, dann der zweite Messbereich 302 und als letztes der Hauptmessbereich 303 den Kontrastmittelbolus zumindest teilweise aufweisen.

Der erste Messbereich 301 weist eine erste Strecke 311 auf, welche ein erstes Ende 311.1 und ein zweites Ende 311.2 aufweist. Das erste Ende 311.1 weist einen ersten Punkt P1 und das zweite Ende 311.2 weist einen zweiten Punkt P2 auf. In dieser Ausgestaltung entspricht das erste Ende 311.1 dem ersten Punkt P1 und das zweite Ende 311.2 dem zweiten Punkt P2.

Der erste Kontrastierungszeitpunkt K1 wird am ersten Punkt P1 im ersten Messbereich 301 und der zweite Kontrastierungszeitpunkt K2 am zweiten Punkt P2 im ersten Messbereich 301 erfasst. Der erste Kontrastierungszeitpunkt K1 und der zweite Kontrastierungszeitpunkt K2 entsprechen jeweils dem Zeitpunkt, an welchem das durch den injizierten Kontrastmittelbolus verstärkte Signal einen Schwellwert 401 jeweils am ersten Punkt P1 und am zweiten Punkt P2 übersteigt. Der Betrag der Differenz zwischen dem ersten Kontrastierungszeitpunkt K1 und dem zweiten Kontrastierungszeitpunkt K2 entspricht der ersten Laufzeit 311.L.

Die erste Strecke 301 ist in dieser Ausgestaltung entlang einer Gefäßstruktur 12.g des Patienten 12 festgelegt, welche mittels der Übersichtsmessdaten erfasst wird. Die erste Strecke 301 weist eine messbare erste Länge 311.S auf.

Eine Flussgeschwindigkeit 311.F des injizierten Kontrastmittelbolus kann durch die erste Strecke 311.S und durch die erste Laufzeit 311.L berechnet werden.

Das Festlegen des zumindest einen Messparameters der angiographischen Messung umfasst insbesondere das Durchführen einer zweiten zeitaufgelösten Messung mittels der Magnetresonanzanlage 11 in dem festgelegten zweiten Messbereich 302, wobei zweite zeitaufgelöste Messdaten generiert werden, das Erfassen des injizierten Kontrastmittelbolus in dem zweiten Messbereich 302 mittels der zweiten zeitaufgelösten Messdaten und das Festlegen eines Messstartzeitpunkts der angiographischen Messung abhängig von dem in dem zweiten Messbereich 302 erfassten injizierten Kontrastmittelbolus.

Fig. 3 zeigt weiterhin den Teilbereich 306 des Hauptmessbereichs 303. Die zweite Strecke 312 weist weiterhin ein erstes Ende 312.1 und ein zweites Ende 312.2 auf, wobei der zweite Messbereich 302 das erste Ende 12.1 und der Hauptmessbereich 303, insbesondere der Teilbereich 306 das zweite Ende 312.2 aufweisen. Wenn beispielsweise der Teilbereich 306 eine besonders interessante Information der angiographischen Messung aufweist, kann mittels der ermittelten zweiten Strecke 312, insbesondere wenn das zweite Ende 312.2 der zweiten Strecke 312 im Teilbereich 306 liegt, und der im ersten Messbereich 301 ermittelten Flussgeschwindigkeit 311.F die zweite Laufzeit 312.L berechnet werden.

Vorzugweise wird ein Mittelzeitpunkt der angiographischen Messung im Hauptmessbereich 303 derart abhängig von der zweiten Laufzeit 312.L festgelegt, dass ein Zentralbereich eines k-Raums des Hauptmessbereichs 303 aufgenommen wird, wenn das zweite Ende 312.2 der zweiten Strecke 312 den injizierten Kontrastmittelbolus aufweist.

Fig. 4 zeigt das Erfassen des Kontrastmittelbolus in einem kontrastierten Gefäß schematisch. Mittels der ersten zeitaufgelösten Messung wird eine Serie an ersten zeitaufgelösten Messdaten zu N Zeitpunkten T₁, T₂, ... T_{N-1} generiert. Fig. 4 zeigt einen Ausschnitt der Messdaten, beispielsweise das Signal in einem oder in einer Menge an Pixeln der Übersichtsbilder über die Zeit. Das Erfassen des injizierten Kontrastmittelbolus entspricht dem Erfassen, wann das Signal, insbesondere die Kontrastierung durch den injizierten Kontrastmittelbolus den Schwellwert 401 überschreitet. Der erste Kontrastierungszeitpunkt K1 entspricht dem Zeitpunkt, wenn das Signal den Schwellwert 401 überschreitet.

## Patentansprüche

1. Verfahren zur Durchführung einer angiographischen Messung von einem Hauptmessbereich (303) eines Patienten (12) mittels einer Magnetresonanzanlage (11), umfassend die folgenden Schritte:
- Durchführen zumindest einer Übersichtsmessung mittels der Magnetresonanzanlage (11), wobei Übersichtsmessdaten generiert werden,
- Festlegen des Hauptmessbereichs (303) und eines ersten Messbereichs (301), welcher sich vom Hauptmessbereich (303) unterscheidet, mittels der Übersichtsmessdaten,
- Durchführen einer ersten zeitaufgelösten Messung mittels der Magnetresonanzanlage (11) in dem festgelegten ersten Messbereich (301), wobei erste zeitaufgelöste Messdaten generiert werden,
- Erfassen eines injizierten Kontrastmittelbolus in dem festgelegten ersten Messbereich (301) mittels der ersten zeitaufgelösten Messdaten,
- Ermitteln einer Flussgeschwindigkeit (311.F) des erfassten injizierten Kontrastmittelbolus,
- Festlegen zumindest eines Messparameters der angiographischen Messung gemäß der ermittelten Flussgeschwindigkeit (311.F) und
- Durchführung der angiographischen Messung des Hauptmessbereichs (303) des Patienten (12) mit dem festgelegten zumindest einen Messparameter in der Magnetresonanzanlage (11).

2. Verfahren nach Anspruch 1, wobei das Ermitteln der Flussgeschwindigkeit (311.F) des injizierten Kontrastmittelbolus umfasst:
- Festlegen einer ersten Strecke (311, 311.S) in dem ersten Messbereich (301),
- Ermitteln einer ersten Laufzeit (311.L) des im ersten Messbereich erfassten injizierten Kontrastmittelbolus auf der ersten Strecke (311) und
- Berechnen der Flussgeschwindigkeit (311.F) unter Verwendung der ersten Strecke (311, 311.S) und der ersten Laufzeit (311.L).

3. Verfahren nach Anspruch 2, wobei die erste Strecke (311, 311.S) entlang einer mittels der Übersichtsmessdaten erfassten Gefäßstruktur (12.g) des Patienten (12) festgelegt wird.

4. Verfahren nach einem der vorhergehenden Ansprüche, wobei das Festlegen des zumindest einen Messparameters der angiographischen Messung umfasst:
- Festlegen einer Messzeitdauer der angiographischen Messung in Abhängigkeit von der ermittelten Flussgeschwindigkeit (311.F).

5. Verfahren nach einem der vorhergehenden Ansprüche, wobei mittels der Übersichtsmessdaten ein zweiter Messbereich (302), welcher sich vom ersten Messbereich (301) und vom Hauptmessbereich (303) unterscheidet, festgelegt wird.

6. Verfahren nach Anspruch 5, wobei der erste Messbereich (301) relativ zum Hauptmessbereich (303) distal gelegen ist und der zweite Messbereich (302) relativ zum Hauptmessbereich (303) proximal gelegen ist.

7. Verfahren nach Anspruch 5 oder 6, wobei der erste Messbereich (301) und/oder der zweite Messbereich (302) abhängig vom Hauptmessbereich (303) automatisch festgelegt werden.

8. Verfahren nach einem der Ansprüche 5-7, wobei ein maximales Gesichtsfeld (17) der Magnetresonanzanlage (11) den ersten Messbereich (301), den zweiten Messbereich (302) und den Hauptmessbereich (303) aufweist.

9. Verfahren nach einem der Ansprüche 5-8, wobei das Festlegen des zumindest einen Messparameters der angiographischen Messung umfasst:
- Durchführen einer zweiten zeitaufgelösten Messung mittels der Magnetresonanzanlage (11) in dem festgelegten zweiten Messbereich (302), wobei zweite zeitaufgelöste Messdaten generiert werden,
- Erfassen des injizierten Kontrastmittelbolus in dem zweiten Messbereich (302) mittels der zweiten zeitaufgelösten Messdaten und
- Festlegen eines Messstartzeitpunkts der angiographischen Messung abhängig von dem in dem zweiten Messbereich (302) erfassten injizierten Kontrastmittelbolus.

10. Verfahren nach einem der Ansprüche 5-9, wobei der zumindest eine Messparameter der angiographischen Messung festgelegt wird unter Verwendung einer zweiten Laufzeit (312.L) des injizierten Kontrastmittelbolus,
wobei eine zweite Strecke (312, 312.S) zwischen dem zweiten Messbereich (302) und dem Hauptmessbereich (303) festgelegt wird und
die zweite Laufzeit (312.L) des injizierten Kontrastmittelbolus entlang der zweiten Strecke (312, 312.S) mittels der in dem ersten Messbereich (301) ermittelten Flussgeschwindigkeit (311.F) des injizierten Kontrastmittelbolus berechnet wird.

11. Verfahren nach Anspruch 10, wobei die zweite Strecke (312, 312.S) zwischen dem zweiten Messbereich (302) und einem Teilbereich (306) des Hauptmessbereichs (303) mittels der Übersichtsmessdaten ermittelt wird.

12. Verfahren nach Anspruch 10 oder 11, wobei das Festlegen des zumindest einen Messparameters der angiographischen Messung unter Verwendung der zweiten Laufzeit (312.L) des injizierten Kontrastmittelbolus umfasst:
- Festlegen eines Mittelzeitpunkts der angiographischen Messung, welcher einem Erfassen eines Zentralbereichs eines k-Raums des Hauptmessbereichs entspricht, abhängig von der zweiten Laufzeit (312.L).

13. Verfahren nach einem der Ansprüche 10-12, wobei die zweite Strecke (312) entlang einer mittels der Übersichtsmessdaten erfassten Gefäßstruktur (12.g) des Patienten (12) festgelegt wird.

14. Magnetresonanzanlage (11), umfassend eine Planungseinheit (20), eine Steuereinheit (14) und eine Messeinheit (18), wobei die Magnetresonanzanlage (11) dazu ausgebildet ist, ein Verfahren nach einem der vorhergehenden Ansprüche auszuführen.

15. Computerprogrammprodukt, welches direkt in einen Speicher einer programmierbaren Steuereinheit einer Magnetresonanzanlage (11) ladbar ist, mit Programmcode-Mitteln, um ein Verfahren nach einem der Ansprüche 1-13 auszuführen, wenn das Computerprogrammprodukt in der Steuereinheit (14) der Magnetresonanzanlage (11) ausgeführt wird.
